# EUROPEAN PATENT SPECIFICATION

(11) **EP 2 832 363 B1**
(45) Date of publication and mention of the grant of the patent: **19.09.2018**
(21) Application number: 13769431.1
(22) Date of filing: 27.03.2013
(51) Int. Cl.: A61K 38/17, A61K 38/16, A61K 39/395, A61K 48/00, A61P 35/00, A61K 39/00

(54) **EPITOPES OF EPIDERMAL GROWTH FACTOR RECEPTOR SURFACE ANTIGEN AND USE THEREOF**
EPITOPE DES EPIDERMALEN WACHSTUMSFAKTOR-REZEPTOR-OBERFLÄCHENANTIGENS UND VERWENDUNG DAVON
ÉPITOPES D'UN ANTIGÈNE DE SURFACE DE RÉCEPTEUR DE FACTEUR DE CROISSANCE ÉPIDERMIQUE ET LEUR UTILISATION

(30) Priority: 27.03.2012 US 201261616073 P
(43) Date of publication of application: 04.02.2015
(73) Proprietor: Green Cross Corporation, Giheung-gu, Yongin-si Gyeonggi-do 446-770 (KR); Mogam Biotechnology Research Institute, Giheung-gu, Yongin-si Gyeonggi-do 446-770 (KR)
(72) Inventor: KIM, Se-Ho, Yongin-si Gyeonggi-do 446-799 (KR); HONG, Kwang-Won, Yongin-si Gyeonggi-do 446-770 (KR); CHANG, Ki Hwan, Yongin-si Gyeonggi-do 446-799 (KR); KIM, Min-Soo, Yongin-si Gyeonggi-do 446-799 (KR); LEE, Mi-Jung, Yongin-si Gyeonggi-do 446-799 (KR); WON, Jong-Hwa, Yongin-si Gyeonggi-do 446-799 (KR); HUR, Min-Kyu, Yongin-si Gyeonggi-do 446-799 (KR); CHO, Hyun-soo, Seoul 120-749 (KR); YOO, Ji-Ho, Seoul 120-749 (KR)
(74) Representative: Grünecker Patent- und Rechtsanwälte PartG mbB
(86) International application number: PCT/KR2013/002550
(87) International publication number: WO 2013/147509

(56) References cited:
- US-A- 5 459 061
- US-A1- 2005 255 555
- US-A1- 2008 253 993
- US-A1- 2009 123 937
- US-B2- 7 449 559
- US-B2- 7 628 986
- BARON A T ET AL: "MONOCLONAL ANTIBODIES SPECIFIC FOR PEPTIDE EPITOPES OF THE EPIDERMAL GROWTH FACTOR RECEPTOR'S EXTRACELLULAR DOMAIN", HYBRIDOMA, LIEBERT, NEW YORK, NY, US, vol. 16, no. 3, 1 June 1997 (1997-06-01), pages 259-271, XP001087533, ISSN: 0272-457X
- HONG K W ET AL: "A novel anti-EGFR monoclonal antibody inhibiting tumor cell growth by recognizing different epitopes from cetuximab", JOURNAL OF BIOTECHNOLOGY, ELSEVIER SCIENCE PUBLISHERS, AMSTERDAM, NL, vol. 145, no. 1, 1 January 2010 (2010-01-01), pages 84-91, XP026798366, ISSN: 0168-1656 [retrieved on 2009-10-12]

<110> Green Cross Corporation Mogam Biotechnology Research Institute
<120> Epitope and it's use of Epidermal Growth Factor Receptor
<130> PP-1202
<150> 61/616, 073
   <151> 2012-03-27
<160> 22
<170> KopatentIn 2.0
<210> 1
   <211> 621
   <212> PRT
   <213> human
<400> 1
<210> 2
   <211> 7
   <212> PRT
   <213> human
<400> 2
   Arg Gly Asp Ser Phe Thr His
   1               5
<210> 3
   <211> 9
   <212> PRT
   <213> human
<400> 3
   Arg Gly Asp Ser Phe Thr His Thr Pro
   1               5
<210> 4
   <211> 29
   <212> DNA
   <213> Artificial Sequence
<220>
   <223> pCTCON-For
<400> 4
   cggctagcct ggaggaaaag aaagtttgc 29
<210> 5
   <211> 27
   <212> DNA
   <213> Artificial Sequence
<220>
   <223> pCTCON-Rev
<400> 5
   cgacgcgttg gacgggatct taggccc 27
<210> 6
   <211> 33
   <212> DNA
   <213> Artificial Sequence
<220>
   <223> R353G-For
<400> 6
   ctgccggtgg catttggcgg tgactccttc aca 33
<210> 7
   <211> 33
   <212> DNA
   <213> Artificial Sequence
<220>
   <223> R353G-Rev
<400> 7
   tgtgaaggag tcaccgccaa atgccaccgg cag 33
<210> 8
   <211> 33
   <212> DNA
   <213> Artificial Sequence
<220>
   <223> F357G-For
<400> 8
   tttaggggtg actccggcac acatactcct cct 33
<210> 9
   <211> 33
   <212> DNA
   <213> Artificial Sequence
<220>
   <223> F357G-Rev
<400> 9
   aggaggagta tgtgtgccgg agtcacccct aaa 33
<210> 10
   <211> 33
   <212> DNA
   <213> Artificial Sequence
<220>
   <223> H359G-For
<400> 10
   ggtgactcct tcacaggcac tcctcctctg gac 33
<210> 11
   <211> 33
   <212> DNA
   <213> Artificial Sequence
<220>
   <223> H359G-Rev
<400> 11
   gtccagagga ggagtgcctg tgaaggagtc acc 33
<210> 12
   <211> 45
   <212> DNA
   <213> Artificial Sequence
<220>
   <223> R353G,F357G-For
<400> 12
   ctgccggtgg catttggcgg tgactccggg acacatactc ctcct 45
<210> 13
   <211> 45
   <212> DNA
   <213> Artificial Sequence
<220>
   <223> R353G,F357G-Rev
<400> 13
   aggaggagta tgtgtcccgg agtcaccgcc aaatgccacc ggcag 45
<210> 14
   <211> 39
   <212> DNA
   <213> Artificial Sequence
<220>
   <223> F357G,H359G-For
<400> 14
   tttaggggtg actccggcac agggactcct cctctggac 39
<210> 15
   <211> 39
   <212> DNA
   <213> Artificial Sequence
<220>
   <223> F357G,H359G-Rev
<400> 15
   gtccagagga ggagtccctg tgccggagtc acccctaaa 39
<210> 16
   <211> 51
   <212> DNA
   <213> Artificial Sequence
<220>
   <223> R353G,H359G-For
<400> 16
   ctgccggtgg catttggcgg tgactccttc acaggtactc ctcctctgga c 51
<210> 17
   <211> 51
   <212> DNA
   <213> Artificial Sequence
<220>
   <223> R353G,H359G-Rev
<400> 17
   gtccagagga ggagtacctg tgaaggagtc accgccaaat gccaccggca g 51
<210> 18
   <211> 51
   <212> DNA
   <213> Artificial Sequence
<220>
   <223> R353G,F357G,H359G-For
<400> 18
   ctgccggtgg catttggcgg tgactccgga acaggtactc ctcctctgga c 51
<210> 19
   <211> 51
   <212> DNA
   <213> Artificial Sequence
<220>
   <223> R353G,F357G,H359G-Rev
<400> 19
   gtccagagga ggagtacctg ttccggagtc accgccaaat gccaccggca g 51
<210> 20
   <211> 25
   <212> DNA
   <213> Artificial Sequence
<220>
   <223> seq-Fl
<400> 20
   atgaaggttt tgattgtctt gttgg 25
<210> 21
   <211> 25
   <212> DNA
   <213> Artificial Sequence
<220>
   <223> seq-Fl
<400> 21
   ccagtgactg ctgccacaac cagtg 25
<210> 22
   <211> 25
   <212> DNA
   <213> Artificial Sequence
<220>
   <223> seq-Fl
<400> 22
   cgtcggcctg aacataacat ccttg 25

## Description

### FIELD OF THE INVENTION

The present invention relates to epitopes on an epidermal growth factor receptor (hereinafter referred to as 'EGFR') and the use thereof. The epitopes provided according to one exemplary embodiment of the present invention are highly conserved and located in a domain closely associated with binding to an epidermal growth factor (hereinafter referred to as 'EGF'). Therefore, vaccine compositions including the epitopes, antibodies against the epitopes, or compositions including the antibodies may efficiently block a signal transduction caused by binding of EGFR to EGF, and thus can be highly valuable and useful in treating various diseases such as cancer. Further, antibodies binding to the epitopes may efficiently inhibit binding of various EGFR ligands such as not only EGF but also transforming growth factor-α (TGF-α), amphiregulin (AR), betacellulin (BTC), epiregulin (EPR) and a heparin-binding EGF-like growth factor (HB-EGF) to EGFR, and thus can be used to treat various diseases caused by activation of EGFR.

Also, the present invention relates to a method of producing an antibody specific to the epitopes.

### BACKGROUND OF THE INVENTION

Immunotherapeutic methods of treating cancer have advantages in that specificity to target diseases in patients is enhanced compared to surgeries, radiation therapies and chemotherapies, thereby enhancing anti-cancer effects and reducing side effects. Tumor-specific monoclonal antibodies have been used as a therapeutic agent which is very useful in treating tumors by targeting certain proteins specifically overexpressed according to various types of cancer to acquire anti-cancer effects.

An epidermal growth factor receptor (EGFR) is a type I membrane protein having a molecular weight of 170 kDa, and is known to be overexpressed in various types of tumors. The EGFR has been studied for a long period of time, and current crystallographic studies of a cellular domain (Garrett TP et al., Cell, 2002, 110: 763-773) and an intracellular kinase domain (Stamos J et al., J. Biol. Chem., 2002, 277: 46265-46272) have been successfully conducted. These studies presented crucial information on the behavior of receptors and ligands thereof. EGFR is a cell surface-associated molecule that is activated through binding of EGF ligands and transforming growth factor-α (TGF-α) thereto. After the binding of the ligands, receptors are dimerized to phosphorylate an intracellular tyrosine kinase domain. As a result, the subsequent signal cascade reactions are activated to induce the growth and proliferation of normal cells and the growth of tumor cells. In particular, since the functions of the tumor cells depend mainly on the EGFR, the receptor has been recognized as a common target for treatment due to the extent of probability of inhibiting the regulatory functions of the EGFR.

The overexpression of the EGFR is, for example, observed in certain types of cancer, such as lung cancer, breast cancer, colon cancer, gastric cancer, brain cancer, bladder cancer, head and neck cancer, ovarian cancer, and prostate cancer. When the binding of EGF to the EGFR is inhibited using antibodies against the EGFR, the growth of cancer cells may be inhibited to treat cancer, which was already experimentally proven using a monoclonal antibody against the EGFR.

Meanwhile, although there have been attempts conducted to elucidate the positions of respective domains of EGFR binding to EGF (S. Yokoyama et al., Cell, 2002, 110: 775-787), it has to be further studied whether an antibody where such EGFR domain is used as an epitope inhibits the activation of an EGFR signal transduction pathway by EGF.

In recent years, cetuximab (C225 antibody, Product name: Erbitux; ImClone, US) used to treat metastatic colorectal cancer in the clinical field is a chimeric antibody which is obtained by binding of a variable region of a mouse antibody to an IgG 1 constant region of a human antibody (having approximately 30% of a mouse amino acid sequence), and thus inhibits the growth of tumor cells and phosphorylation of EGFR by EGF *in vitro,* and suppresses the formation of human tumors in a nude mouse. Also, the antibody was found to have synergism with a certain chemotherapeutic agent to eradicate the human tumors in a xenograft mouse model. However, cetuximab has a problem in that it causes an immune response in patients (approximately 10%), and can only be used as a combination therapy with a chemotherapeutic agent since it does not have a satisfactory therapeutic effect using the stand-alone therapy.

Panitumumab (Product name: Vectibix; Amgen Inc. US) that is another antibody used to treat metastatic colorectal cancer is a fully human antibody which inhibits the growth of tumor cells and phosphorylation of EGFR by EGF *in vitro,* and suppresses the formation of human tumors in a nude mouse. Also, the antibody was found to have synergism with a certain chemotherapeutic agent to eradicate the human tumors in a xenograft mouse model. Panitumumab is different from cetuximab in that it is a fully human antibody, has an antigen binding ability 10 times of cetuximab, reduces the probability of inducing an immune response like cetuximab as an IgG2-type antibody, and exhibits only an inhibitory effect of signal transduction by binding of EGFR to EGF due to limitations of IgG2 although the binding ability was enhanced to reduce the side effects and improve effectiveness. Therefore, it was reported that panitumumab has overall clinical effects similar to cetuximab because it has no antibody-dependent cell cytotoxicity (ADCC) activities among the inhibitory effect of signal transduction and ADCC activities of cetuximab known in the related art.

Also, matuzumab (mAb425) which is being jointly developed by Merck Serono and Takeda Pharmaceuticals does not have a satisfactory therapeutic effect in the stand-alone therapy so far.

Above all, all the conventional antibody therapeutic agents targeting the EGFR are not allowed to be prescribed to treat K-ras mutant colorectal cancer. The data reported in 2009 confirmed that a percentage of patients in which the two antibodies show a therapeutic effect amounts to approximately 21%, and the antibodies show no therapeutic effect in the remaining 79 % of the patients. 43% of the patients group in which the antibodies show no therapeutic effect, i.e., approximately 30% of the overall number of patients, have a K-ras mutant, which belongs to this group of K-ras mutant colorectal cancer. The conventional antibody therapeutic agents, cetuximab and panitumumab, show a therapeutic effect only in approximately 1.5% of K-ras mutant.

Therefore, the demands for novel anti-EGFR antibodies having differentiation and superiority with which the problems and limits of the conventional EGFR antibodies can be overcome by inhibiting the binding of EGFR to EGF more efficiently tend to continuously increase.
Baron et al. (1997), Hybridoma vol. 16, no. 3, 259-271 describes monoclonal antibodies specific for peptide epitopes of the epidermal growth factor receptor's extracellular domain. US 5459061 describes hybridomas producing monoclonal antibodies which specifically bind to continuous epitope on the human EGF receptor and compete with EGF for binding to the EGF receptor.

### SUMMARY OF THE INVENTION

The subject matter of the invention is as set out in the claims.

It is an objective of the present invention to provide an amino acid sequence of RGDSFTH (SEQ ID NO: 2), or an epitope on EGFR including the same, and particularly, an epitope having an amino acid sequence of RGDSFTHTP (SEQ ID NO: 3).

It is another objective of the present invention to provide a method of producing the epitope, a composition for cancer vaccines or a cancer vaccine including the epitope, a method of producing an antibody, which has the ability to specifically bind to the epitope, using the epitope, and a composition or a therapeutic agent for preventing and/or treating cancer including the antibody produced by the method.

Accordingly the invention provides an epitope of an epidermal growth factor receptor (EGFR) consisting of the amino acid sequence of RGDSFTH (SEQ ID NO: 2) or RGDSFTHTP (SEQ ID NO: 3).

The invention further provides a complex in which the above epitope is bound to a carrier, wherein the carrier is preferably at least one selected from the group consisting of a peptide, serum albumin, immunoglobulin, hemocyanin and polysaccharide.

The invention further provides a polynucleotide encoding any of the above epitopes.

The invention further provides a recombinant vector comprising the above polynucleotide.

The invention further provides a recombinant microorganism or virus transformed with the above recombinant vector, wherein the recombinant microorganism or virus is preferably selected from the group consisting of a recombinant E. coli, a recombinant yeast and a recombinant bacteriophage.

The invention further provides a method of preparing the epitope, wherein said epitope consists of the amino acid sequence of SEQ ID NO: 2; or the amino acid sequence of SEQ ID NO: 3, wherein said method comprises culturing the above recombinant microorganism or virus expressing the above recombinant vector.

The invention further comprises a vaccine composition for use in a method of treating cancer, wherein said composition comprises the above epitope, the above complex comprising the epitope, or the above polynucleotide encoding the epitope.

The invention further comprises a method of producing an antibody, preferably a polyclonal or monoclonal antibody, or a fragment thereof, specifically binding to the epitope consisting of the amino acid sequence of SEQ ID NO: 2 or SEQ ID NO: 3, by using the epitope consisting of the amino acid sequence of SEQ ID NO: 2 or the amino acid sequence of SEQ ID NO: 3; a complex comprising the epitope, or a polynucleotide encoding the epitope.

As described above, the present invention provides the epitope or a polynucleotide sequence encoding the epitope. A further disclosure relates to a composition and a kit for diagnosing cancer including the polynucleotide sequence.

### BRIEF DESCRIPTION OF THE DRAWINGS

**FIG. 1** is a diagram showing the binding characteristics of EGFR to a GC1118 antibody in a 3D manner.
**FIG. 2** is a diagram showing the binding characteristics of EGFR to EGF in a 3D manner.
**FIG. 3** is a diagram showing the binding characteristics of the GC1118 antibody to EGFR to which EGF is bound when the GC1118 antibody overlaps the EGFR.
**FIG. 4** is a diagram showing the binding characteristics of EGFR to cetuximab and matuzumab in a 3D manner.
**FIG. 5** is a diagram showing a procedure of synthesizing an EGFR variant gene and a procedure of transforming yeast cells.
**FIG. 6** is a diagram showing that respective EGFR variants are properly synthesized by identifying the amino acid sequence determined from a DNA base sequence.
**FIG. 7** is a diagram showing the expression rates of the respective EGFR variants.
**FIG. 8** is a diagram showing the binding abilities of antibodies against the respective EGFR mutants: (A) GC1118, and (B) cetuximab (control).
**FIG. 9** is a diagram showing the competitive binding abilities of EGFR ligands to GC1118: (A) GC1118, and (B) cetuximab (control).
**FIG. 10** is a diagram showing the inhibitory activities of GC1118 on the induction of cell proliferation.

### DETAILED DESCRIPTION OF THE INVENTION

Hereinafter, the present invention will be described in detail.

The present inventors have elucidated that an amino acid sequence of RGDSFTH (SEQ ID NO: 2) or an amino acid sequence including the same, especially an amino acid sequence represented by RGDSFTHTP (SEQ ID NO: 3), essentially functions in binding to EGF among amino acid sequences of EGFR, and found that antibodies having the amino acid sequence as an epitope very efficiently inhibit the binding of EGFR to EGF, and thus shows superior effects in treating cancer by blocking the signal transduction from the binding. Therefore, the present invention has been completed based on these facts.

The amino acid sequence of RGDSFTH set forth in SEQ ID NO: 2 corresponds to 353^{rd} to 359^{th} amino acid residues of an amino acid sequence of an extracellular domain of EGFR set forth in SEQ ID NO: 1, and the amino acid sequence of RGDSFTHTP set forth in SEQ ID NO: 3 corresponds to 353^{rd} to 361^{st} amino acid residues of the extracellular domain of EGFR set forth in SEQ ID NO: 1.

The amino acid residues present in the amino acid sequence provided in the present invention are represented by their three- or one-letter abbreviations known in the related art. Also, in the present invention, the term "xA" refers to an A^{th} amino acid x of an EGFR sequence set forth in SEQ ID NO: 1, and the term "xAz" means that an A^{th} amino acid x is substituted with z. For example, the term "R353" refers to arginine (Arg) that is the 353^{rd} amino acid residue of the amino acid sequence set forth in SEQ ID NO: 1, and the term "R353G" means that arginine (Arg) that is the 353^{rd} amino acid residue of the amino acid sequence set forth in SEQ ID NO: 1 is substituted with glycine (Gly).

An epitope having the amino acid sequence set forth in SEQ ID NO: 2, the amino acid sequence comprising the amino acid sequence of SEQ ID NO: 2, or the amino acid sequence set forth in SEQ ID NO: 3 may be used in combination with a carrier in order to maintain its own 3D structure or provide efficiency in use as a composition such as a cancer vaccine. The carrier according to one exemplary embodiment of the present invention is biocompatible, and all types of carriers may be used herein as long as they can achieve desired effects. In this case, the carrier may be selected from the group consisting of a peptide, serum albumin, an immunoglobulin, hemocyanin, and a polysaccharide, but the present invention is not limited thereto.

An epitope consisting of the amino acid sequence set forth in SEQ ID NO: 2 or the amino acid sequence set forth in SEQ ID NO: 3 may be used per se, or used as a complex in combination with a carrier. In this case, the epitope or the complex may be used in a cancer vaccine composition. Here, the vaccine composition may further include a pharmaceutically acceptable adjuvant or excipient. Any type of adjuvant may be used as long as they can serve to enhance antibody formation when injected into the body, thereby achieving the objectives of the present invention. In particular, the adjuvant may be at least one selected from the group consisting of an aluminum salt (Al(OH)₃, or AlPO₄), squalene, sorbitane, polysorbate 80, CpG, a liposome, colesterol, monophosphoryl lipid A (MPL), and glucopyranosyl lipid A (GLA), but the present invention is not limited thereto.

A polynucleotide encoding the epitope consisting of the amino acid sequence set forth in SEQ ID NO: 2 or the amino acid sequence set forth in SEQ ID NO: 3 provided in the present invention may be used in the form of a genetic cancer vaccine per se. In this case, the polynucleotide itself may be used without using a delivery system, or may be delivered into the body contained in a viral or non-viral delivery system. Any type of viral or non-viral delivery system may be used as long as they are known to be conventionally available in the art. Specifically, the viral delivery system preferably includes an adenovirus, an adeno-associated virus, a lentivirus, a retrovirus, and the like, and the non-viral vector that may be used herein includes at least one selected from the group consisting of a cationic polymer, a non-ionic polymer, a liposome, a lipid, phospholipid, a hydrophilic polymer, a hydrophobic polymer, and a complex thereof, but the present invention is not limited thereto.

The present invention provides a recombinant vector including the polynucleotide encoding the epitope consisting of the amino acid sequence set forth in SEQ ID NO: 2 or the amino acid sequence set forth in SEQ ID NO: 3, a host cell including the recombinant vector, and a method of preparing the epitope, which consists of the amino acid sequence of SEQ ID NO: 2 or the amino acid sequence of SEQ ID NO: 3, using the recombinant vector or the host cell according to the present invention.

In the present invention, the term "recombinant vector" refers to an expression vector capable of expressing a target protein in a proper host cell, that is, a gene construct including an essential regulatory element operably linked to express a gene insert. In the present invention, the term "operably linked" means that a nucleic acid sequence encoding a desired protein is functionally linked with a nucleic acid expression control sequence to execute general functions. The operable linkage with the recombinant vector may be performed using genetic recombination techniques widely known in the art, and the site-spectific DNA cleavage and ligation may be readily performed using enzymes widely known in the art.

A proper expression vector that can be used in the present invention may include signal sequences for membrane targeting or secretion in addition to expression control elements such as a promoter, an initiation codon, a stop codon, a polyadenylation signal, and an enhancer. The initiation codon and the stop codon are generally considered to be a portion of a nucleotide sequence encoding an immunogenic target protein, and thus should be essentially operated in an individual when a gene construct is administered into the individual, and inserted in-frame with a coding sequence. A common promoter may be constitutive or inducible. There are Lac, Tac, T3, and T7 promoters present in prokaryotic cells, but not limited thereto. There are a β-actin promoter and promoters derived from human hemoglobin, human muscle creatine, and human metallothionein, as well as promoters derived from a simian virus 40(SV40), a mouse mammary tumor virus (MMTV), a human immunodeficiency virus (HIV) (for example, a long terminal repeat (LTR) promoter from HIV), a Moloney virus, a cytomegalovirus (CMV), an Epstein-Barr virus (EBV), and a Rous sarcoma virus (RSV) present in eukaryotic cells, but not limited thereto.

The expression vector may include a selection marker for selecting a host cell containing the vector. The selection marker is used to select cells transformed with the vector. Here, markers giving selectable phenotypes such as drug tolerance, auxotrophy, tolerance to cytotoxic agents, or expression of surface proteins may be used as the selection marker. Since only the cells expressing the selection marker survive in an environment treated with a selective agent, it is possible to select the transformed cells. Also, when the vector is a replicable expression vector, the vector may include a replication origin that is a certain nucleic acid sequence from which a replication is initiated. Various types of vectors such as a plasmid, a virus, and a cosmid may be used as the recombinant expression vector. Any type of the recombinant vector may be used without particular limitation as long as they can function to express a desired gene in various host cells such as prokaryotic cells and eukaryotic cells, and produce a desired protein. However, a vector, which includes a promoter having strong activities and can mass-produce a foreign protein having a similar shape to the wild type while retaining strong expression intensity, may be preferably used as the recombinant vector.

In particular, various combinations of expression hosts/vectors may be employed to express the epitope according to one exemplary embodiment of the present invention, which consists of the amino acid sequence set forth in SEQ ID NO: 2 or the amino acid sequence set forth in SEQ ID NO: 3. The expression vector suitable for eukaryotic hosts may contain regulatory sequence for expression, including without limitation, derived from SV40, a bovine papilloma virus, an adenovirus, an adeno-associated virus, a cytomegalovirus, a lentivirus and a retrovirus. The expression vector that may be used in a bacterial host includes bacterial plasmids obtained from *Escherichia coli,* such as pET, pRSET, pBluescript, pGEX2T, pUC vector, col El, pCR1, pBR322, pMB9 and derivatives thereof; plasmids having a wide host range such as RP4; phage DNAs including various phage lambda derivatives such as λgt10, λgt11 and NM989; and other DNA phages such as M13 and filamentous single-stranded DNA phages. A vector useful for insect cells is pVL941.

The recombinant vector is introduced into host cells to form transformants. The suitable host cells may include prokaryotes such as *E. coli, Bacillus subtilis, Streptomyces* sp., *Pseudomonas* sp., *Proteus mirabilis,* or *Staphylococcus* sp.; fungi such as *Aspergillus* sp.; yeast cells such as *Pichia pastoris, Saccharomyces cerevisiae, Schizosaccharomyces* sp., and *Neurospora crassa;* and other lower eukaryotes, and higher eukaryotic cells such as insect cells. Further, the host cells may be preferably derived from plants, and mammals such as monkey kidney cells (COS7), NSO cells, SP2/0, Chinese hamster ovary (CHO) cells, W138, baby hamster kidney (BHK) cells, MDCK, a myeloma cell line, HuT 78 cells, and HEK293 cells, without limitation. The CHO cells are particularly preferred.

In the present invention, the term "transformation" into the host cells encompasses any method of introducing a nucleic acid sequence into an organism, a cell, a tissue, or an organ, and may be performed using standard techniques suitable for the host cell as known in the art. Such methods include, without limitation, such as electroporation, protoplast fusion, calcium phosphate (CaPO₄) precipitation, calcium chloride (CaCl₂) precipitation, agitation using silicon carbide fibers, *Agrobacteria*-mediated transformation, a PEG method, a dextran sulfate method, a lipofectamine method, and drying/suppression-mediated transformation. The epitope according to one exemplary embodiment of the present invention, which has the amino acid sequence set forth in SEQ ID NO: 2, the amino acid sequence comprising the amino acid sequence of SEQ ID NO: 2 or the amino acid sequence set forth in SEQ ID NO: 3, may be subjected to large-scale production by culturing the transformants expressing the recombinant vector in a nutrient medium. The medium and culture conditions may be properly selected depending upon the host cell. The conditions such as temperature, pH of a medium, and a culture time may be properly adjusted to enable the efficient growth of cells and mass-production of a protein. As described above, the epitope having the amino acid sequence set forth in SEQ ID NO: 2, the amino acid sequence comprising the amino acid sequence of SEQ ID NO: 2 or the amino acid sequence set forth in SEQ ID NO: 3 may be recovered recombinantly from a medium, or a cell lysate, and may be separated and purified using conventional biochemical separation techniques (Sambrook et al., Molecular Cloning: A laborarory Manual, 2nd Ed., Cold Spring Harbor Laboratory Press(1989); Deuscher, M., Guide to Protein Purification Methods Enzymology, Vol. 182. Academic Press. Inc., San Diego, CA (1990)). The biochemical separation techniques that may be used herein may include, without limitation, electrophoresis, centrifugation, gel filtration, precipitation, dialysis, chromatography (ion exchange chromatography, affinity chromatography, immunoabsorbent chromatography, or size exclusion chromatography), isoelectric focusing, and various modified and combined methods thereof.

The present invention provides a method of expressing an epitope, which consists of an amino acid sequence set forth in SEQ ID NO: 2 or an amino acid sequence set forth in SEQ ID NO: 3, on the surface of microorganisms or viruses. In this method, a recombinant vector characterized in that it comprises a sequence encoding an inducible promoter or a signal protein, and various microorganisms or viruses comprising the recombinant vector may be used. The particularly proper microorganisms or viruses include, without limitation, recombinant *E. coli,* recombinant yeasts, and recombinant bacteriophages. Display techniques widely known in the art may be used to express the epitope consisting of an amino acid sequence as set forth in SEQ ID NO: 2 or SEQ ID NO: 3 on the surface of the microorganism or virus. Particularly, a method of binding a polynucleotide sequence encoding the epitope, which consists of the amino acid sequence set forth in SEQ ID NO: 2 or the amino acid sequence set forth in SEQ ID NO: 3, to a sequence encoding a promoter or a signal protein to induce the expression of the epitope on the surface of microbial or viral cells, or a method of deleting a portion of a gene encoding a protein originally expressed on the surface of the microbial or viral cells and inserting a polynucleotide sequence encoding the epitope, which consists of the amino acid sequence set forth in SEQ ID NO: 2 or the amino acid sequence set forth in SEQ ID NO: 3, into the partially deleted gene, may be used herein, but not limited thereto. The epitope expressed on the surfaces of the microorganisms or viruses, which consists of the amino acid sequence set forth in SEQ ID NO: 2 or the amino acid sequence set forth in SEQ ID NO: 3, may be separated and purified per se, and may be used for the purpose of specific use according to one exemplary embodiment of the present invention, and may be used for panning an antibody specifically binding to the epitope, which consists of the amino acid sequence set forth in SEQ ID NO: 2 or the amino acid sequence set forth in SEQ ID NO: 3, when expressed on the surface of the microorganisms or viruses, to obtain the antibody.

Also, the present invention provides an epitope consisting of an amino acid sequence set forth in SEQ ID NO: 2 or an amino acid sequence set forth in SEQ ID NO: 3, an antibody specifically binding to the epitope, which consists of the amino acid sequence set forth in SEQ ID NO: 2 or the amino acid sequence set forth in SEQ ID NO: 3, using a complex including the epitope or a polynucleotide encoding the epitope, or a method of producing a fragment of such an antibody. Such an antibody may be a polyclonal antibody, or a monoclonal antibody, and the fragment of the antibody falls within the scope of the present invention as long as it retains characteristics to bind to the epitope, which consists of the amino acid sequence set forth in SEQ ID NO: 2 or the amino acid sequence set forth in SEQ ID NO: 3. In particular, the antibody or its fragment according to one exemplary embodiment of the present invention includes, without limitation, single-chain antibodies, diabodies, triabodies, tetrabodies, Fab fragments, F(ab')₂ fragments, Fd, scFv, domain antibodies, bispecific antibodies, minibodies, scAb, IgD antibodies, IgE antibodies, IgM antibodies, IgG1 antibodies, IgG2 antibodies, IgG3 antibodies, IgG4 antibodies, derivatives of constant regions of the antibodies, and artificial antibodies based on protein scaffolds, as long as they have a binding activity to the epitope, which consists of the amino acid sequence set forth in SEQ ID NO: 2 or the amino acid sequence set forth in SEQ ID NO: 3. Also, antibodies having mutations in variable regions thereof are encompassed within the scope of the present invention as long as they retain their characteristics according to one exemplary embodiment of the present invention. By way of example, the mutations may include conservative substitutions of amino acids in the variable regions. The conservative substitution refers to a substitution of an original amino acid sequence with another amino acid residue having a similar characteristic. For example, lysine, arginine, and histidine residues are similar in terms of having a basic side chain. Also, aspartic acid and glutamic acid residues are similar in terms of having an acidic side chain. Also, glycine, asparagine, glutamine, serine, threonine, tyrosine, cysteine, and tryptophan residues are similar in terms of having a non-charged, polar side chain, and alanine, valine, leucine, threonine, isoleucine, proline, phenylalanine, and methionine residues are similar in terms of having a non-polar side chain. Further, tyrosine, phenylalanine, tryptophan, and histidine residues are similar in terms of having an aromatic side chain. Therefore, it is apparent to those skilled in the art that the amino acid substitutions among the group of the amino acids having the similar characteristics as described above will not cause any change in the characteristics. As a result, a method of producing an antibody having mutations by conservative substitutions in a variable region thereof falls within the scope of the present invention as long as it retains its characteristics.

The antibody binding to the epitope according to the present invention, which consists of the amino acid sequence set forth in SEQ ID NO: 2 or the amino acid sequence set forth in SEQ ID NO: 3, may be obtained using methods widely known in the art to which the present invention belongs. Specifically, the antibody may be prepared by inoculating a non-human animal with an epitope, which consists of the amino acid sequence set forth in SEQ ID NO: 2 or the amino acid sequence set forth in SEQ ID NO: 3, a complex including the epitope, or a polynucleotide encoding the epitope and producing and panning an antibody specifically binding to the epitope, which consists of the amino acid sequence set forth in SEQ ID NO: 2 or the amino acid sequence set forth in SEQ ID NO: 3, from the inoculated animal.

In this case, the animal is preferably an animal transformed to produce an antibody having the same sequence as a human-derived sequence, especially a transformed rat. Here, a fully human antibody having reduced immunogenicity may be prepared using the transformed rat in accordance with methods disclosed in US Patent Nos. 5,569,825; 5,633,425; and 7,501,552. When the non-human animal is not transformed to produce the antibody having the same sequence as the human-derived sequence, a humanization or deimmunization step may be further performed on the antibody obtained from the non-human animal so that the antibody becomes suitable for a treatment use in the body, as described in the methods disclosed in US Patent Nos. 5,225,539; 5,859,205; 6,632,927; 5,693,762; 6,054,297; and 6,407,213; and WO 1998/52976. Specifically, the humanization or deimmunization step may include a CDR grafting step of engrafting a CDR sequence of the antibody produced from the animal into the framework (FR) of a human antibody, and a CDR-walking step of substituting, inserting or deleting at least one amino acid sequence in order to further enhance affinity or reduce immunogenicity.

When a full-length EGFR rather than the epitope, which consists of the amino acid sequence set forth in SEQ ID NO: 2 or the amino acid sequence set forth in SEQ ID NO: 3, the complex including the epitope, or the polynucleotide encoding the epitope, is used as an immunogen, a method of panning an antibody, which includes primarily panning antibodies having binding capacity to the EGFR and panning the antibody specifically recognizing the epitope, which consists of the amino acid sequence set forth in SEQ ID NO: 2 or the amino acid sequence set forth in SEQ ID NO: 3, among the primarily panned antibodies, may also be used. In this case, a method of panning an antibody, which include inducing mutations in a crucial binding site of the epitope, which consists of the amino acid sequence set forth in SEQ ID NO: 2 or the amino acid sequence set forth in SEQ ID NO: 3, and panning the antibodies having lost or reduced binding capacity to the EGFR due to the mutations in the crucial binding site of the epitope, which consists of the amino acid sequence set forth in SEQ ID NO: 2 or the amino acid sequence set forth in SEQ ID NO: 3, among the primarily panned antibodies binding to the EGFR, may also be used herein.

Also, a human antibody binding to the epitope set forth in SEQ ID NO: 2 or SEQ ID NO: 3 may be produced and panned using a display technique widely known in the art to which the present invention belongs. The display technique is preferably at least one selected from the group consisting of phage display, yeast display, bacteria display, and ribosome display techniques, but the present invention is not limited thereto. The preparation and display of a library may be easily performed as disclosed in US Patent Nos. 5,733,743; 7063943; 6172197; 6,348,315; and 6,589,741. Particularly, the library used in the display is preferably designed to have a sequence of the human-derived antibody. Specifically, the method is characterized in that it includes panning only antibodies specifically binding to the epitope, which consists of the amino acid sequence set forth in SEQ ID NO: 2 or the amino acid sequence set forth in SEQ ID NO: 3, using the epitope, which consists of the amino acid sequence set forth in SEQ ID NO: 2 or the amino acid sequence set forth in SEQ ID NO: 3, or the complex including the epitope.

Finally, the present invention provides an epitope consisting of an amino acid sequence set forth in SEQ ID NO: 2, or an amino acid sequence set forth in SEQ ID NO: 3 a complex including the epitope, or a composition for cancer vaccines including a polynucleotide encoding the epitope.

The present invention will be described in further detail through the exemplary embodiments below.

### Example 1: Identification of binding structure of EGFR to Fab of GC1118

To identify a binding structure of EGFR to an fragment antigen-binding (Fab) of GC1118 (see KR 10-2011-0034914 A) developed by a group of researchers of the present invention, the X-ray diffraction data of a crystal structure of an EGFR/GC1118 Fab composite was obtained using the BL26B2 beamline (Spring-8 Institution, Japan), and indexed and scaled using HKL2000 software (HKL Research Inc., US), and an early electron density map of the EGFR/GC1118 composite was then obtained using a molecular replacement (MR) method. To employ the MR method, the data on a 3D structure of a protein having a structure similar to that of the EGFR/target antibody composite was needed. In this case, a structure of an EGFR/cetuximab composite (PDB ID: 1YY9) was used as the structure for the EGFR/GC1118 composite. The information on an early phase of the EGFR/GC1118 composite was obtained using Molrep program (http://www.ysbl.york.ac.uk/∼alexei/molrep.html), and a model building task was performed using a crystallographic object-oriented toolkit (COOT: http://www.biop.ox.ac.uk/coot/), based on the obtained information on the early phase. Then, a refinement task was completed using Refmac5 (http://www.ccp4.ac.uk/html/refmac5.html) software and python-based hierarchical environment for integrated xtallography (PHENIX: http:// www.phenix-online.org/) software.

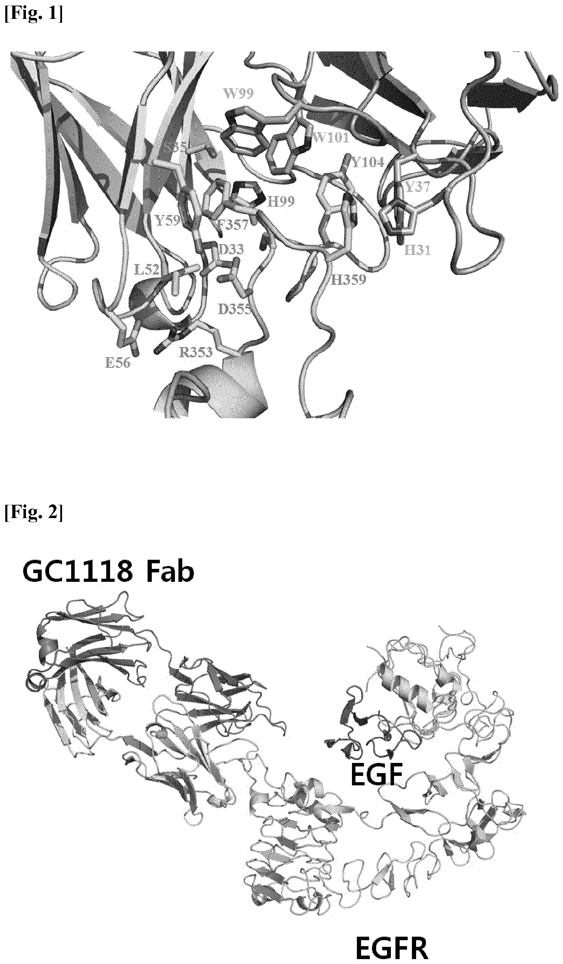

As a result, it was revealed that the epitope on EGFR of GC1118 was positioned in a 3^{rd} domain of EGFR, and particularly the epitope was a short loop region of the 3^{rd} domain, which consists of 9 amino acid residues spanning from the 353^{rd} to 361^{st} amino acid residues of the amino acid sequence (see SEQ ID NO: 1 and FIG. 1). This loop region protrudes outwards, and is surrounded by CDR of the antibody. In particular, the amino acid residues playing a crucial role in binding between GC1118 and EGFR are F357 and H359 of the EGFR. The two amino acid residues are surrounded by amino acid residues of a CDR region of the GC1118 antibody, and function to form hydrophobic bonds and hydrogen bonds with the amino acid residues of the CDR region in order to firmly maintain the binding between EGFR and GC1118. Also, R353 contributes to further binding to the antibody via ionic bonds to a variable region (VH) of a heavy chain of the GC1118 antibody (see FIG. 1).

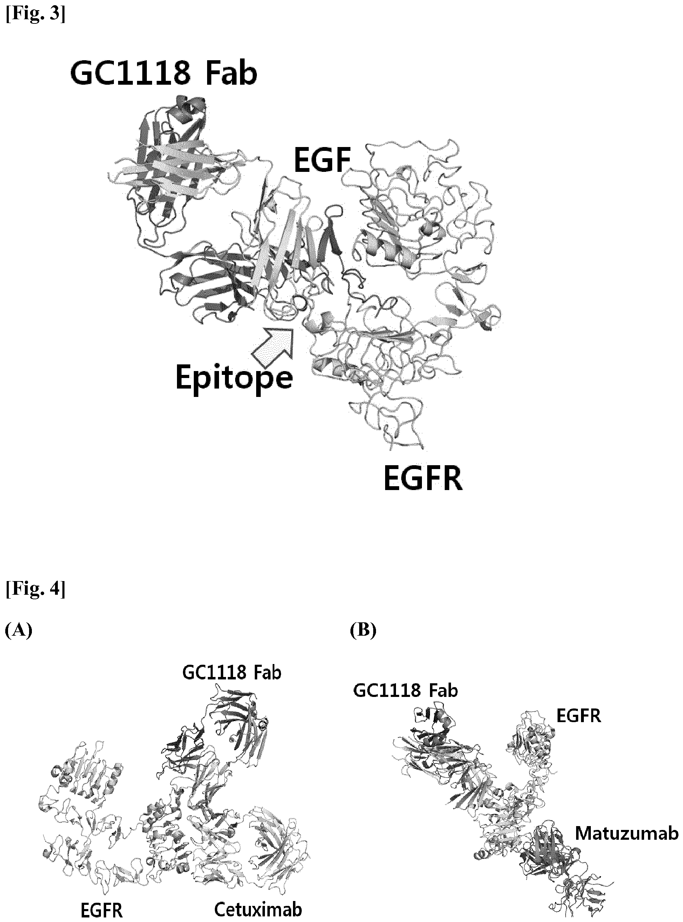

Meanwhile, a region to which EGF binds is positioned between 1^{st} and 3^{rd} domains of EGFR. In this case, EGF first binds to the 1^{st} domain of EGFR (see FIG. 2) to induce a structural change of the EGFR domain, and then binds to the 3^{rd} domain (see FIG. 3). In this case, the important EGFR amino acid residue participating in the binding to EGF is D355 of the 3^{rd} domain (Ferguson et al., Molecular Cell, 2003, 11:507-517). Here, the amino acid residue is present in a loop containing R353, F357 and H359 which are the epitopes of GC1118. That is, since the epitopes on EGFR to which GC1118 binds are present in the same region as the region to which EGF binds, the antibody binding to the epitopes of GC1118 may directly inhibit the binding of EGF to the 3^{rd} domain of EGFR. This is further confirmed by comparison with the structure of EGFR bound to EGF. Accordingly, when the structure of EGFR activated through the binding of EGF overlaps that of GC1118, it could be seen that the VH region of GC1118 and EGF overlapped each other (see FIG. 3).

On comparison with the binding of currently used cetuximab to EGFR, cetuximab had epitopes positioned in the 3^{rd} domain of EGFR, but the binding sites of cetuximab were widely dispersed on a surface of the 3^{rd} domain. Therefore, some of the epitopes had an indirect influence on the binding of EGF (see FIG. 4A). Matuzumab which was another antibody also had epitopes positioned in the 3^{rd} domain of EGFR, but the epitopes of matuzumab were present at different positions than the epitopes according to one exemplary embodiment of the present invention, and positioned in a region other than the region to which EGF was bound so that the epitopes of matuzumab were not able to directly hinder the binding of EGF (see FIG. 4B).

### Example 2: Verification of epitopes on EGFR of GC1118 using yeast cell surface expression method

To verify the epitopes (R353, F357, and H359 amino acid residues) of EGFR against GC1118 found through a structure model, the corresponding amino acid residues were substituted with glycine having no binding reactivity to produce the variants, and then expressed on surfaces of yeast cells. Thereafter, the binding of GC1118 to EGFR variants in which the epitopes were expressed on the surfaces of the yeast cells was confirmed using flow cytometry (BD FACSCalibur™, US). As a result, it was confirmed that the binding affinity to GC1118 was reduced in all of the EGFR variants.

### 2.1 Construction of EGFR variants

EGFR variants were constructed by substituting one or more of R353, F357 and H359 of the amino acid sequence of EGFR (see SEQ ID NO: 1) with glycine using an overlapping PCR method in which a gene sequence encoding an extracellular domain of human EGFR was used as a template. The results are listed in the following Table 1.

**Table 1**

| **EGFR variants substituted with glycine (Glv)** | | | | |
|---|---|---|---|---|
| EGFR variants | Amino acid substitution | Details | | |
| | | Position | Before substitution | After substitution |
| Variant 1 | R353G | 353 | Arg | Gly |
| Variant 2 | F357G | 357 | Phe | Glv |
| Variant 3 | H359G | 359 | His | Gly |
| Variant 4 | R353G + F357G | 353 | Arg | Gly |
| | | 357 | Phe | Gly |
| Variant 5 | F357G + H359G | 357 | Phe | Gly |
| | | 359 | His | Gly |
| Variant 6 | R353G + H359G | 353 | Arg | Gly |
| | | 359 | His | Gly |
| Variant 7 | R353G + F357G + H359G | 353 | Arg | Gly |
| | | 357 | Phe | Gly |
| | | 359 | His | Gly |

### 2.1.1 PCR reaction for synthesis of EGFR variants

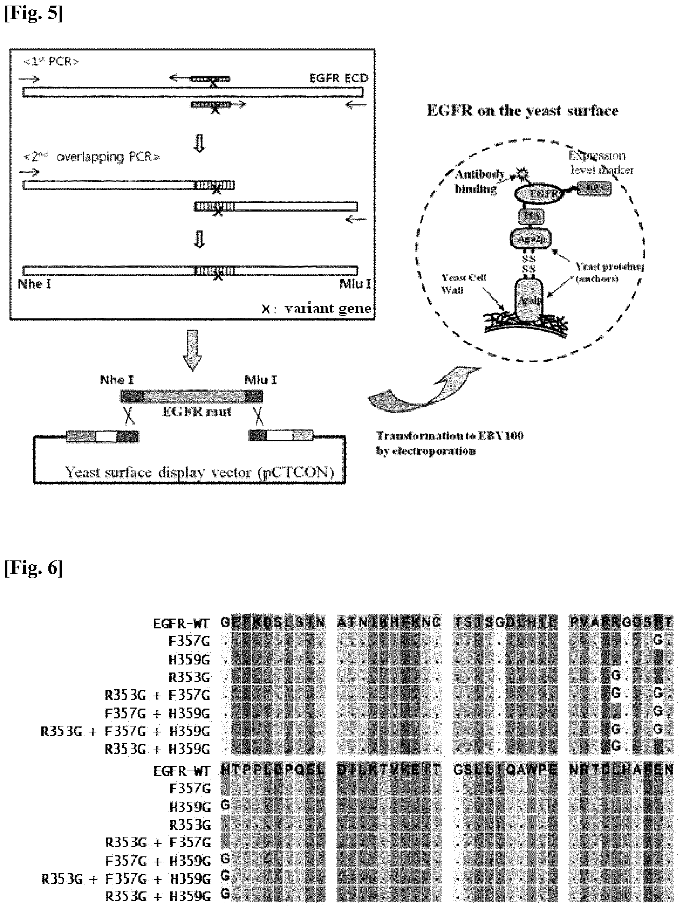

Two gene fragments (approximately 1,150 bp and 720 bp) commonly including each of the EGFR variants were synthesized by performing a PCR reaction using AccuPower™ TLA PCR PreMix (Bioneer, Korea) including a gene sequence of the human EGFR as the template, and a set of primers (see Table 2) designed to induce mutation of an EGFR gene. The two synthesized gene fragments were electrophoresed in 1% agarose gel, and the DNA of each of the gene fragments was purified using a Qiagen kit (Qiagen 28706, Germany). Subsequently, an overlapping PCR reaction was performed using the DNA of the set of the two purified gene fragments as one template and primers having amino acid sequences set forth in SEQ ID NOS: 4 to 19 (see Table 2), thereby synthesizing final EGFR variant genes (see FIG. 5). The synthesized genes were cleaved using restriction enzymes NheI/MluI (New England BioLabs, US). The term "For" following the primer names as listed in Table 2 represents a forward primer, and the term "Rev" represents a reverse primer.

**Table 2**

| **Primers used in PCR reaction to construct EGFR variants** | | | |
|---|---|---|---|
| **Primer name** | **Target variants** | **Sequence** | **SEQ ID NO** |
| pCTCON-For | Control | 5'-CGGCTAGCCTGGAGGAAAAGAAAGTTTGC-3' | 4 |
| pCTCON-Rev | | 5'-CGACGCGTTGGACGGGATCTTAGGCCC-3' | 5 |
| R353G-For | Variant 1 | 5'-CTGCCGGTGGCATTTGGCGGTGACTCCTTCACA-3' | 6 |
| R353G-Rev | | | 7 |
| F357G-For | Variant 2 | 5'-TTTAGGGGTGACTCCGGCACACATACTCCTCCT-3' | 8 |
| F357G-Rev | | | 9 |
| H359G-For | Variant 3 | 5'-GGTGACTCCTTCACAGGCACTCCTCCTCTGGAC-3' | 10 |
| H359G-Rev | | | 11 |
| R353G,F357G-For | Variant 4 | | 12 |
| R353G,F357G-Rev | | | 13 |
| F357G,H359G-For | Variant 5 | | 14 |
| F357G,H359G-Rev | | | 15 |
| R353G,H359G-For | Variant 6 | | 16 |
| R353G,H359G-Rev | | | 17 |
| R353G,F357G, H359G-For | Variant 7 | | 18 |
| R353G,F357G, H359G-Rev | | | 19 |

### 2.1.2 Ligation and transformation of EGFR variants

A yeast surface expression vector pCTCON (Boder, E.T. et al., Nat Biotechnol. 1997, 15(6):553-7) was cleaved using restriction enzymes NheI/MluI, electrophoresed in 1% agarose gel, and purified using a Qiagen kit. The EGFR variant DNAs prepared in Example 2.1.1 were mixed with pCTCON, and a T4 DNA ligase (New England BioLabs, US) was added thereto, and reacted at 25 °C for 2 hours. The ligation mixture was transformed with *E. coli* XL1-blue cells (Stratagene, US) through electroporation using Gene Pulser (Bio-Rad Laboratories, Inc., US), and incubated for an hour in a total of 2 mL of a medium. Thereafter, the transformed cells were spread on an LB-agar plate supplemented with carbenicillin (Sigma, US). Subsequently, the cells were incubated overnight at 37 °C.

### 2.1.3 Base sequencing of EGFR variants

Colonies on the plate cultured in Example 2.1.2 were incubated overnight in 2 mL of an LB medium supplemented with 50 µg/mL of carbenicillin, and a recombinant plasmid was then separated using a Qiagen plasmid minikit (Qiagen 27106, Germany) to determine DNA base sequences of the EGFR variants inserted into the plasmid. Primers having sequences set forth in SEQ ID NOS: 20 to 22 were used as the sequencing primers used for the base sequencing (see Table 3), and the base sequencing was performed and analyzed by Genotech Corporation (Daejeon, Republic of Korea) according to a known method. The base sequence of human EGFR was used as the control. After the DNA base sequences of the EGFR variants were determined, the determined DNA base sequences were translated into amino acid sequences using a web-based program (www.expasy.org: DNA to Protein translate tool) which translates a base sequence into an amino acid sequence. The translation results are shown in FIG. 6. Then, it was confirmed that all the EGFR variant genes listed in Table 1 were correctly inserted into the pCTCON vectors.

**Table 3**

| **Primers used in sequencing reaction** | | |
|---|---|---|
| Types of primers | Sequence | SEQ ID NO |
| seq-F1 | 5'- ATGAAGGTTTTGATTGTCTTGTTGG | 20 |
| seq-F1 | 5'-CCAGTGACTGCTGCCACAACCAGTG | 21 |
| seq-F1 | 5'- CGTCGGCCTGAACATAACATCCTTG | 22 |

### 2.2 Analysis of expression of EGFR variants on yeast cell surfaces and binding to the antibody

### 2.2.1 Procedure of expressing EGFR variants on surfaces of yeast cells

A EBY100 (*S. cerevisiae*) yeast strain was transformed with the pCTCON-EGFR verified in Example 2.1 using Gene Pulser from Bio-Rad Laboratories, Inc. (FIG. 1). The transformed colonies were incubated at 30 °C for 20 hours in a selective medium, an SD-CAA liquid medium (20 g/L glucose, 6.7 g/L yeast nitrogen base without amino acids, 5.4 g/L Na₂HPO₄, 8.6 g/L NaH₂PO₄H₂O, and 5 g/L casamino acids). Thereafter, the expression of the EGFR variant on surfaces of yeast cells was induced by incubating the transformed colonies at 30 °C for 20 hours in a selective medium, an SD-CAA liquid medium (20 g/L galactose, 6.7 g/L yeast nitrogen base without amino acids, 5.4 g/L Na₂HPO₄, 8.6 g/L NaH₂PO₄H₂O, and 5 g/L casamino acids).

### 2.2.2 Determination of expression of EGFR variants on surfaces of yeast cells

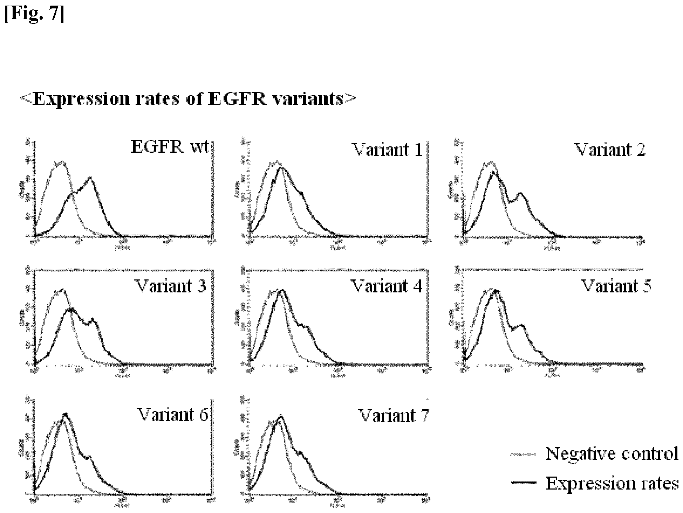

The expression of the EGFR variants on the surfaces of the yeast cells was confirmed using BD FACS Calibur™ (Becto Dickinson). The yeast cells expressed at a density of approximately 1×10⁷ cells/ml were reacted with anti-c-myc 9E10 mAb (dilution of 1:100) in 0.1 ml of phosphate buffered saline (PBSB including 1 mg/ml BSA; pH 7.4) (at 25 °C for 30 minutes), and washed with PBSB. The yeast cells were secondarily reacted with R-phycoerythrin conjugated goat anti-mouse IgG (dilution of 1:25) on ice for 15 minutes, washed with PBSB, and then analyzed using BD FACS Calibur™. From the analysis results, it was revealed that the EGFR variants were well expressed on the surfaces of the yeast cells (see FIG. 7).

### 2.2.3 Analysis of binding between EGFR variants and GC1118 using flow cytometry

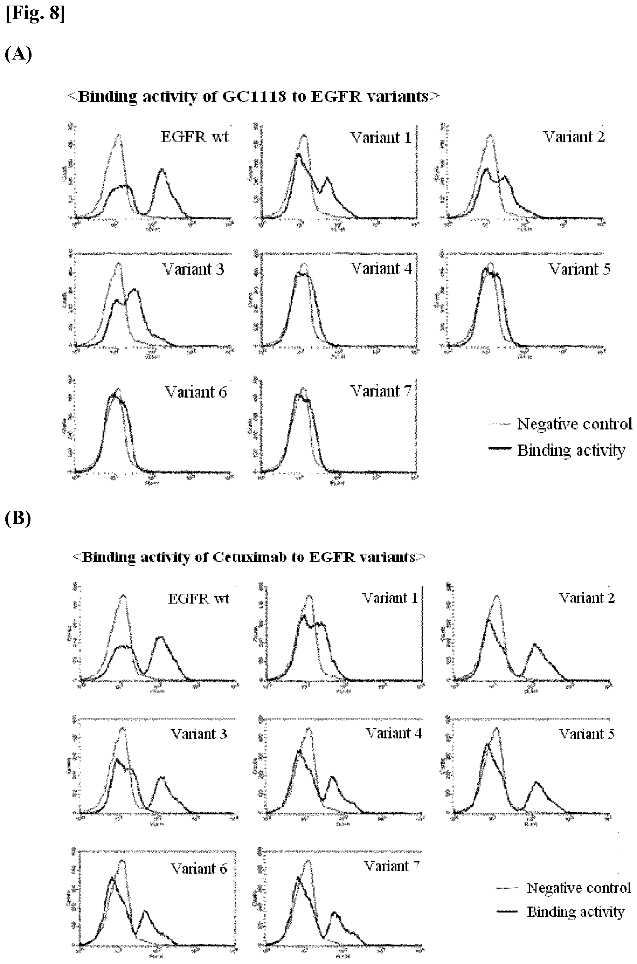

The binding between GC1118 and the EGFR variants expressed on the surfaces of the yeast cells was confirmed using BD FACS Calibur™ (Becton Dickinson). Cetuximab was used as a control antibody. The yeast cells expressed at a density of approximately 1×10⁷ cells/ml were reacted with 1 µg of each of the antibodies in 0.1 ml of PBSB (including 1 mg/ml BSA; pH 7.4) (at 25 °C for 30 minutes), and washed with PBSB. The yeast cells were secondarily reacted with FITC conjugated anti-human IgG (dilution of 1:50) on ice for 15 minutes, washed with PBSB, and then measured for mean fluorescence intensity using BD FACS Calibur™. The measurement results are listed in Table 4 and shown in FIG. 8. As seen from Table 4, it was proven that the binding of GC1118 was inhibited in the EGFR variants in which the epitopes(R353, F357 and H359 according to one exemplary embodiment of the present invention through the structural analysis) were substituted with glycine, and thus the substituted amino acid residues were the epitopes of GC1118.

**Table 4**

| **Binding of antagonist to EGFR variants** | | | |
|---|---|---|---|
| **EGFR Variants** | **Expression Level (%)** | **Antagonist binding activity (%)** | |
| | | **GC1118** | **Cetuximab** |
| EGFR (wild type) | 100 | 100 | 100 |
| Variant 1 (R353G) | 66.5 | 30.4 | 23.8 |
| Variant 2 (F357G) | 91.3 | 26.1 | 88.3 |
| Variant 3 (H359G) | 89.3 | 34.1 | 82.9 |
| Variant 4 (R353G+F357G) | 71.4 | 14.1 | 34.8 |
| Variant 5 (F357G+H359G) | 74.4 | 15 | 77.3 |
| Variant 6 (R353G+H359G) | 68 | 13.7 | 35.2 |
| Variant 7 (R353G+F357G+H359G) | 69.2 | 13.61 | 42.6 |

### Example 3: Analysis of competitive binding of GC1118 and ligands to EGFR

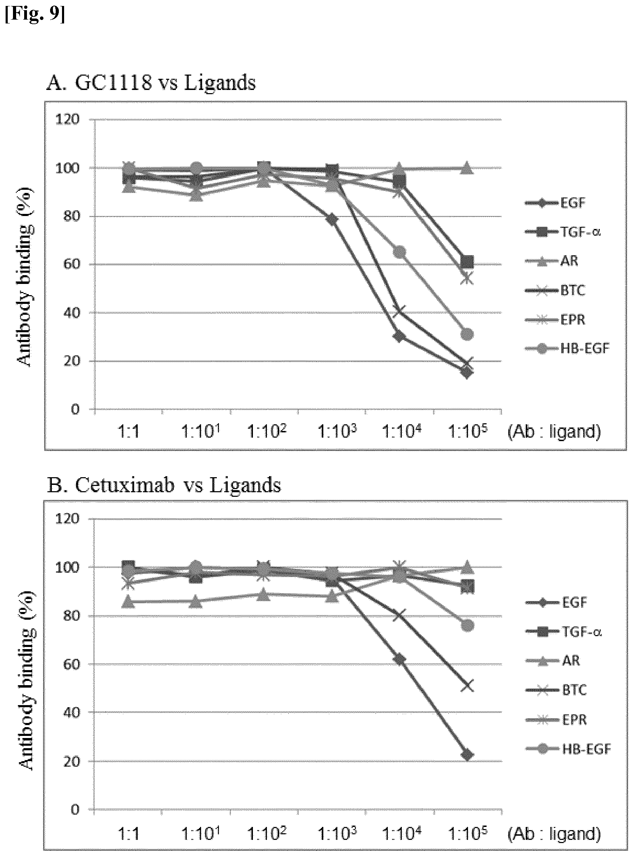

To determine whether GC1118 and six ligands listed in Table 5 had identical or similar binding sites for EGFR, a competitive binding test was performed. Cetuximab was used as a control antibody. First, a predetermined concentration of an antibody (1.5 nM) and various concentrations of a ligand were reacted together on a plate coated with 2 µg/ml of EGFR. In this case, the ligand was present at a concentration so that the molar ratio of the antibody and the ligand amounted to 1:1, 1:10, 1:10², 1:10³, 1:10⁴, and 1:10⁵. The resulting mixture was reacted at room temperature for 30 minutes, and then washed five times with PBST (including 0.05% Tween20; pH 7.4). Then, the mixture was secondarily reacted with anti-human IgG (Fc specific) Peroxidase conjugated (Sigma A0170, US) at room temperature for 30 minutes, and washed five times with PBST. Subsequently, 100 µL of a 3,3',5,5'-tetramethylbenzidine (TMB) Microwell Peroxidase Substrate (KPL 50-76-03, US) solution was added to each well, and then measured for O.D values at 405 nm. It was noticed that two reactants (the antibody and the ligand) competed against each other when the binding affinity of GC1118 to EGFR was reduced with an increasing concentration of the ligand, indicating that the two reactants had the identical epitopes to the EGFR, or were a very close match. The competition between the antibody and the ligands is shown in FIG. 9 and listed in Table 6. As a result, it could be seen that GC1118 has superior ability to inhibit the binding of EGF, TGF-α, BTC, EPR, and HB-EGF, compared to cetuximab.

**Table 5**

| **EGFR ligands used** | |
|---|---|
| **Human EGFR ligands** | **Details** |
| EGF | Epidermal growth factor |
| TGF-α | Transforming growth factor-α |
| AR | Amphiregulin |
| BTC | β-Cellulin |
| EPR | Epiregulin |
| HB-EGF | Heparin-binding EGF-like growth factor |

**Table 6**

| **Competitive binding capacities of EGFR ligands to GC1118** | | | | |
|---|---|---|---|---|
| | Antibody binding (%) | | | |
| | GC1118 | | Cetuximab | |
| Antibody : ligand (molar ratio) | 1:10⁴ | 1:10⁵ | 1:10⁴ | 1:10⁵ |
| EGF | 30.2 | 15.2 | 61.8 | 22.5 |
| TGF-α | 94.1 | 61.1 | 96.5 | 92.4 |
| AR | 99.6 | 99.9 | 96.6 | 99.9 |
| BTC | 40.4 | 18.9 | 80.2 | 51.1 |
| EPR | 90.0 | 54.4 | 99.9 | 91.5 |
| HB-EGF | 65.1 | 31.2 | 96.2 | 76.1 |

### Example 4: Inhibitory effect on cell proliferation induced by EGFR ligands

To compare an inhibitory effect on GC1118 of the present invention and cetuximab on cancer cell proliferation induced by EGFR ligands, a colorectal cancer cell line, LS174T (ATCC, CL-188), was spread on a 96-well microplate (Nunc) so that the number of cells amounted to 3,000 cells/ml. After one day, a culture medium was removed, and culture media including no bovine serum albumin were treated with GC1118 and the control antibody, cetuximab, at concentrations of 0.1, 1, and 10 µg/ml. At the same time as the antibody treatment, the culture media were treated with EGFR ligands by type at concentrations (EGF; 50 ng/ml, TGF-α; 100 ng/ml, amphiregulin; 100 ng/ml, epiregulin; 300 ng/ml, HB-EGF; 50 ng/ml, and β-cellulin; 100 ng/ml), and then incubated at 37 °C for 3 days in a CO₂ incubator. To measure a proliferation level of cancer cells after 3 days, 40 µl of an MTS solution (CellTiter 96 Aqueous One solution Reagent, Promega) was added to the cancer cells incubated in the 96-well plate, reacted at 37 °C for 3 hours in a CO₂ incubator, and then measured for O.D values at 490 nm.

As a result, it could be seen that GC1118 much more efficiently inhibited the proliferation of the cancer cells induced by the EGF, TGF-α, HB-EGF, and BTC ligands, compared to cetuximab, which indicated that these results corresponded to those of Example 3.

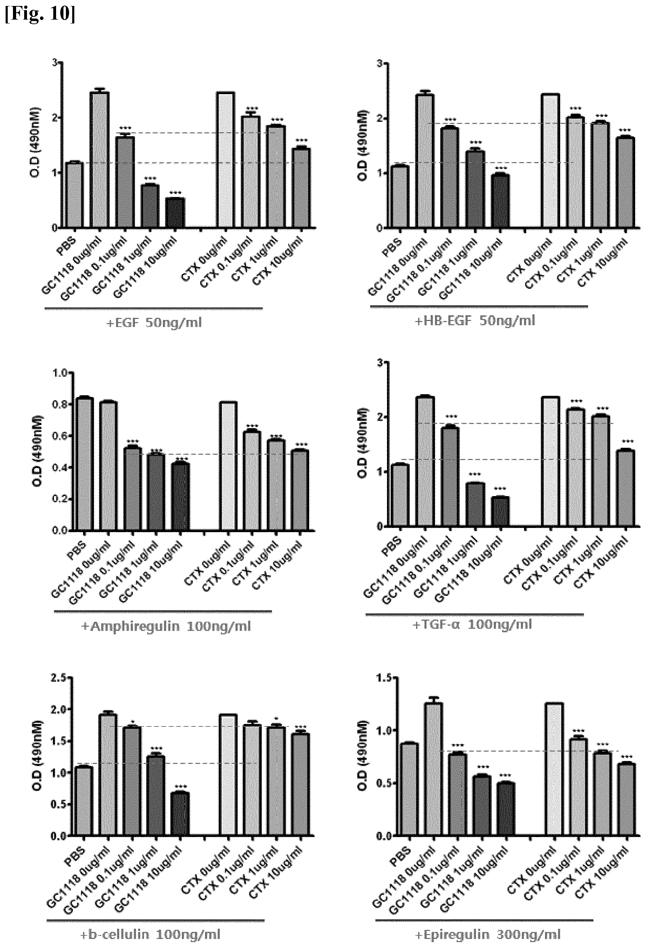

From the above results, it was revealed that the antibodies having the epitopes according to one exemplary embodiment of the present invention showed superior inhibitory effect on the cell proliferation since the antibodies had different epitopes than cetuximab, and thus had an ability to inhibit the ligand binding (see FIG. 10). In particular, the antibodies binding to the epitopes according to one exemplary embodiment of the present invention are able to be used to treat various diseases such as cancer developed by the activation of EGFR due to the binding of not only EGF but also the other EGFR ligands since the antibodies efficiently inhibits the binding of the various EGFR ligands such as not only EGF but also TGF-α, AR, BTC, EPR and HB-EGF to EGFR.

### INDUSTRIAL APPLICABILITY

The epitopes on EGFR provided according to one exemplary embodiment of the present invention are highly conserved and located in a domain closely associated with binding to EGF. Therefore, the compositions including antibodies against the epitopes, or the vaccine compositions including the epitopes can efficiently block a signal transduction caused by binding of EGR to EGFR, and thus can be highly valuable and useful in treating various diseases such as cancer.
## Claims

1. An epitope of an epidermal growth factor receptor (EGFR) consisting of the amino acid sequence of RGDSFTH (SEQ ID NO: 2) or RGDSFTHTP (SEQ ID NO: 3).

2. A complex in which the epitope of claim 1 is bound to a carrier, wherein the carrier is preferably at least one selected from the group consisting of a peptide, serum albumin, immunoglobulin, hemocyanin and polysaccharide.

3. A polynucleotide encoding any of the epitopes of claim 1.

4. A recombinant vector comprising the polynucleotide of claim 3.

5. The recombinant vector of claim 4, which comprises a promoter inducing an expression of the epitope on the surface of a microorganism cell or virus, or a nucleotide sequence encoding a signal protein.

6. A recombinant microorganism or virus transformed with the recombinant vector of claim 4 or 5, wherein the recombinant microorganism or virus is preferably selected from the group consisting of a recombinant E. coli, a recombinant yeast and a recombinant bacteriophage.

7. A method of preparing the epitope, wherein said epitope consists of the amino acid sequence of SEQ ID NO: 2; or the amino acid sequence of SEQ ID NO: 3, wherein said method comprises culturing the recombinant microorganism or virus of claim 6 expressing the recombinant vector of claim 4 or claim 5.

8. A vaccine composition for use in a method of treating cancer, wherein said composition comprises the epitope of claim 1, the complex of claim 2 comprising the epitope, or the polynucleotide of claim 3 encoding the epitope.

9. The vaccine composition for use as in claim 8, further comprising a pharmaceutically acceptable adjuvant improving the formation of an antibody when injected into a body, wherein the adjuvant is preferably at least one selected from the group consisting of aluminum salts (Al(OH)₃, AlPO₄), squalene, sorbitane, polysorbate 80, CpG, liposome, cholesterol, MPL (monophosphoryl lipid A) and GLA (glucopyranosyl lipid A).

10. The vaccine composition for use as in claim 8, wherein the polynucleotide is included in a pharmaceutically acceptable carrier, preferably wherein the pharmaceutically acceptable carrier is a viral or a non-viral carrier.

11. A method of producing an antibody, preferably a polyclonal or monoclonal antibody, or a fragment thereof, specifically binding to the epitope consisting of the amino acid sequence of SEQ ID NO: 2 or SEQ ID NO: 3,
by using the epitope consisting of the amino acid sequence of SEQ ID NO: 2 or the amino acid sequence of SEQ ID NO: 3; a complex comprising the epitope, or a polynucleotide encoding the epitope.

12. The method of claim 11, which comprises the steps of:
administering to a non-human animal the epitope, the complex comprising the epitope, or the polynucleotide encoding the epitope; and
panning the antibody from the animal which specifically binds to the epitope.

13. The method of claim 12, which further comprises the step of performing
• humanization, preferably comprising grafting CDR sequence of the antibody produced from the animal to framework (FR) of a human antibody; or
• deimmunization.

14. The method of claim 13, which further comprises the step of performing substitution, insertion or deletion of at least one amino acid for improving affinity or reducing immunogenicity.

15. The method of claim 12, wherein the non-human animal is a transgenic animal which can produce an antibody having an identical amino acid sequence to that of human antibody, wherein the transgenic animal is preferably a transgenic mouse.

16. The method of claim 11, which employs a display technique wherein the display technique is preferably selected from the group comprising phage display, bacteria display, and ribosome display, and wherein the display preferably employs a library designed to comprise a sequence of human-originated antibody.

17. The method of claim 16, which comprises
panning the antibody specifically binding to the epitope, or to the complex comprising the epitope.

## Patentansprüche

1. Epitop eines epidermalen Wachstumsfaktorrezeptors (EGFR), bestehend aus der Aminosäuresequenz RGDSFTH (SEQ ID NO: 2) oder RGDSFTHTP (SEQ ID NO: 3).

2. Komplex, in welchem das Epitop nach Anspruch 1 an einen Carrier gebunden ist, wobei es sich bei dem Carrier vorzugsweise um mindestens einen handelt, ausgewählt aus der Gruppe, bestehend aus einem Peptid, Serumalbumin, Immunglobulin, Hämocyanin und Polysaccharid.

3. Polynucleotid, welches eines der Epitope nach Anspruch 1 kodiert.

4. Rekombinanter Vektor, umfassend das Polynucleotid nach Anspruch 3.

5. Rekombinanter Vektor nach Anspruch 4, welcher einen Promotor, der eine Expression des Epitops auf der Oberfläche einer Mikroorganismenzelle oder eines Virus induziert, oder eine Nucleotidsequenz, die ein Signalprotein kodiert, umfasst.

6. Rekombinanter Mikroorganismus oder rekombinantes Virus, transformiert mit dem rekombinanten Vektor nach Anspruch 4 oder 5, wobei der rekombinante Mikroorganismus oder das rekombinante Virus vorzugsweise aus der Gruppe ausgewählt ist, bestehend aus einem rekombinanten E. coli, einer rekombinanten Hefe und einem rekombinanten Bakteriophagen.

7. Verfahren zum Herstellen des Epitops, wobei das Epitop aus der Aminosäuresequenz der SEQ ID NO: 2 oder der Aminosäuresequenz der SEQ ID NO: 3 besteht, wobei das Verfahren das Züchten des rekombinanten Mikroorganismus oder Virus nach Anspruch 6, welcher bzw. welches den rekombinanten Vektor nach Anspruch 4 oder Anspruch 5 exprimiert, umfasst.

8. Impfstoffzusammensetzung zur Verwendung bei einem Verfahren zum Behandeln von Krebs, wobei die Zusammensetzung das Epitop nach Anspruch 1, den Komplex nach Anspruch 2, welcher das Epitop umfasst, oder das Polynucleotid nach Anspruch 3, welches das Epitop kodiert, umfasst.

9. Impfstoffzusammensetzung zur Verwendung wie in Anspruch 8, ferner umfassend ein pharmazeutisch verträgliches Adjuvans, das die Bildung eines Antikörpers verbessert, wenn es in einen Körper injiziert wird, wobei es sich bei dem Adjuvans vorzugsweise um mindestens eines handelt, ausgewählt aus der Gruppe, bestehend aus Aluminiumsalzen (Al(OH)₃, AlPO₄), Squalen, Sorbitan, Polysorbat 80, CpG, Liposom, Cholesterin, MPL (Monophosphoryllipid A) und GLA (Glucopyranosyllipid A).

10. Impfstoffzusammensetzung zur Verwendung wie in Anspruch 8, wobei das Polynucleotid in einem pharmazeutisch verträglichen Carrier eingeschlossen ist, wobei es sich bei dem pharmazeutisch verträglichen Carrier um einen viralen oder einen nichtviralen Carrier handelt.

11. Verfahren zum Herstellen eines Antikörpers, vorzugsweise eines polyklonalen oder monoklonalen Antikörpers, oder eines Fragments davon, der bzw. das spezifisch an das aus der Aminosäuresequenz der SEQ ID NO: 2 oder SEQ ID NO: 3 bestehende Epitop bindet,
unter Verwendung des aus der Aminosäuresequenz der SEQ ID NO: 2 oder der Aminosäuresequenz der SEQ ID NO: 3 bestehenden Epitops, eines Komplexes, welcher das Epitop umfasst, oder eines Polynucleotids, welches das Epitop kodiert.

12. Verfahren nach Anspruch 11, welches die Schritte umfasst:
Verabreichen des Epitops, des Komplexes, welcher das Epitop umfasst, oder des Polynucleotids, welches das Epitop kodiert, an ein nichtmenschliches Tier, und
Anreichern des Antikörpers, der spezifisch an das Epitop bindet, von dem Tier durch Panning.

13. Verfahren nach Anspruch 12, welches ferner den Schritt umfasst,
• Humanisierung, vorzugsweise umfassend, die CDR-Sequenz des produzierten Antikörpers von dem Tier auf die Framework (FR) eines humanen Antikörpers zu verpflanzen, oder
• Deimmunisierung.
durchzuführen.

14. Verfahren nach Anspruch 13, welches ferner den Schritt umfasst, zum Verbessern der Affinität oder Reduzieren der Immunogenität eine Substitution, Insertion oder Deletion von mindestens einer Aminosäure durchzuführen.

15. Verfahren nach Anspruch 12, wobei es sich bei dem nichtmenschlichen Tier um ein transgenes Tier handelt, das einen Antikörper produzieren kann, der eine mit der eines humanen Antikörpers identische Aminosäuresequenz aufweist, wobei es sich bei dem transgenen Tier vorzugsweise um eine transgene Maus handelt.

16. Verfahren nach Anspruch 11, welches eine Display-Technik einsetzt, wobei die Display-Technik vorzugsweise aus der Gruppe ausgewählt ist, umfassend Phagendisplay, Bakteriendisplay und Ribosomendisplay, und wobei das Display vorzugsweise eine Bibliothek einsetzt, die entwickelt wurde, um eine Sequenz eines Antikörpers humanen Ursprungs zu umfassen.

17. Verfahren nach Anspruch 16, welches umfasst, den Antikörper, der spezifisch an das Epitop oder an den das Epitop umfassenden Komplex bindet, durch Panning anzureichern.

## Revendications

1. Épitope d'un récepteur de facteur de croissance épidermique (EGFR) constitué de la séquence d'acides aminés de RGDSFTH (SEQ ID n° : 2) ou RGDSFTHTP (SEQ ID n° : 3).

2. Complexe dans lequel l'épitope selon la revendication 1 est lié à un support, le support étant préférablement au moins l'un sélectionné dans le groupe constitué d'un peptide, d'une albumine sérique, d'une immunoglobuline, d'une hémocyanine et d'un polysaccharide.

3. Polynucléotide codant pour n'importe lequel des épitopes selon la revendication 1.

4. Vecteur recombinant comprenant le polynucléotide selon la revendication 3.

5. Vecteur recombinant selon la revendication 4, qui comprend un promoteur d'induction d'une expression de l'épitope sur la surface d'une cellule de micro-organisme ou d'un virus, ou une séquence nucléotidique codant pour une protéine signal.

6. Micro-organisme recombinant ou virus transformé avec le vecteur recombinant selon la revendication 4 ou 5, le micro-organisme recombinant ou le virus étant préférablement sélectionné dans le groupe constitué *d'E. coli* recombinant, d'une levure recombinante et d'un bactériophage recombinant.

7. Procédé de préparation de l'épitope, ledit épitope étant constitué de la séquence d'acides aminés de SEQ ID n° : 2 ; ou de la séquence d'acides aminés de SEQ ID n° : 3, ledit procédé comprenant la culture du micro-organisme recombinant ou du virus selon la revendication 6 exprimant le vecteur recombinant selon la revendication 4 ou la revendication 5.

8. Composition de vaccin pour l'utilisation dans un procédé de traitement du cancer, ladite composition comprenant l'épitope selon la revendication 1, le complexe selon la revendication 2 comprenant l'épitope, ou le polynucléotide selon la revendication 3 codant pour l'épitope.

9. Composition de vaccin pour l'utilisation selon la revendication 8, comprenant en outre un adjuvant pharmaceutiquement acceptable améliorant la formation d'un anticorps lorsqu'il est injecté dans un corps, l'adjuvant étant préférablement au moins l'un sélectionné dans le groupe constitué des sels d'aluminium (Al(OH)₃, AlPO₄), du squalène, du sorbitane, du polysorbate 80, du CpG, du liposome, du cholestérol, du MPL (monophosphoryl lipide A) et du GLA (glucopyranosyl lipide A).

10. Composition de vaccin pour l'utilisation selon la revendication 8, le polynucléotide étant compris dans un support pharmaceutiquement acceptable, préférablement le support pharmaceutiquement acceptable étant un support viral ou non viral.

11. Procédé de production d'un anticorps, préférablement d'un anticorps polyclonal ou monoclonal, ou son fragment, se liant spécifiquement à l'épitope constitué de la séquence d'acides aminés de SEQ ID n° : 2 ou SEQ ID n° : 3,
en utilisant l'épitope constitué de la séquence d'acides aminés de SEQ ID n° : 2 ou la séquence d'acides aminés de SEQ ID n° : 3 ; un complexe comprenant l'épitope, ou un polynucléotide codant pour l'épitope.

12. Procédé selon la revendication 11, qui comprend les étapes de :
administration à un animal non humain de l'épitope, le complexe comprenant l'épitope, ou le polynucléotide codant pour l'épitope ; et
adhérence sur plastique de l'anticorps provenant de l'animal qui se lie spécifiquement à l'épitope.

13. Procédé selon la revendication 12, qui comprend en outre l'étape d'exécution
• de l'humanisation, préférablement comprenant le greffage de la séquence CDR de l'anticorps produit à partir de l'animal au cadre (FR) d'un anticorps humain ; ou
• de désimmunisation.

14. Procédé selon la revendication 13, qui comprend en outre l'étape d'exécution de la substitution, de l'insertion ou de la délétion d'au moins un acide aminé pour améliorer l'affinité ou réduire l'immunogénicité.

15. Procédé selon la revendication 12, l'animal non humain étant un animal transgénique qui peut produire un anticorps ayant une séquence d'acides aminés identique à celle de l'anticorps humain, l'animal transgénique étant préférablement une souris transgénique.

16. Procédé selon la revendication 11, qui utilise une technique d'affichage dans lequel la technique d'affichage est préférablement sélectionnée dans le groupe comprenant l'affichage de phage, l'affichage de bactéries, et l'affichage de ribosome, et l'affichage utilisant préférablement une bibliothèque conçue pour comprendre une séquence d'un anticorps provenant de l'être humain.

17. Procédé selon la revendication 16, qui comprend
l'adhérence sur plastique de l'anticorps se liant spécifiquement à l'épitope, ou au complexe comprenant l'épitope.
